# EUROPEAN PATENT APPLICATION

(11) **EP 2 278 335 A1**
(43) Date of publication of application: **26.01.2011**
(21) Application number: 09165930.0
(22) Date of filing: 20.07.2009
(51) Int. Cl.: G01N 33/68

(54) **Novel method for determination of a pregnancy status of a mammal**

(71) Applicant: Forschungsverbund Berlin E.V., 12489 Berlin (DE)
(72) Inventor: Dehnhard, Martin, 12559 Berlin (DE); Rohleder, Marlies, 10247 Berlin (DE)
(74) Representative: Gulde Hengelhaupt Ziebig & Schneider

(57) **Abstract**

The present invention provides a method for determination of a pregnancy status of a mammal, comprising: i) providing faeces material of a mammal; and ii) measuring the concentration of at least one PGF_{2α} metabolite in said faeces material.

## Description

### BACKGROUND OF THE INVENTION:

Animal husbandry, maintenance and successful breeding are the major challenges for the animal's conservation. Breeding also demands practical methods for monitoring endocrine activity including reliable pregnancy tests.

Currently, there are numerous methods available that can be employed in veterinary, zoo animal or wild animal medicine.

One option is to test for the concentration of progesterone (P4) or metabolites thereof in blood, urine or other body fluids. The detection of sustained elevated levels of progesterone indicate pregnancy. One problem associated with the measurement of progesterone levels is that, for exact measurement, it is necessary to take blood samples. The taking of a blood sample usually requires to anaesthetise the animal, which is a burden for the person collecting the blood and is always connected with a risk for the animal. Furthermore, the testing for progesterone levels in carnivores is not informative in the first half of pregnancy. Although in the second half of pregnancy the progesterone levels are significantly elevated, in a number of carnivores testing for progesterone does not allow to discriminate between real pregnancy and naturally occurring pseudopregnancy.

Another option is to test for the level and/or presence of the placental proteo-hormone relaxin. The protein relaxin is detectably in blood and is excreted via the urine. Since the concentration of relaxin in urine is very low and the test is not very sensitive, it is necessary to collect and concentrate the urine of a particular animal, before an informative measurement can be conducted. Such collection is sometimes difficult because urine is draining away in a short period of time and because, especially when numerous animals are living together in a group, disposed urine cannot easily be attributed to a particular animal. In such a case, it may be necessary to anaesthetise the animal which is complicating the whole procedure and is always connected with a risk for the animal. Furthermore the polypeptide sequence of the hormone relaxin is species specific, so that it will not be possible to provide a test system which will work for all or at least a large number of different animal species.

Of course, it is also possible to use ultrasound investigations to test for the pregnancy status of an animal. Such investigations are time consuming and require an expensive ultrasound equipment the use of which may be limited to specific locations and may not be available in the environment where the animals to be tested are usually held. Since body movement hampers ultrasound image acquisition, it may be necessary to anaesthetise the animal which, again, is complicating the whole procedure and is always connected with a risk for the animal.

A further option is to test for prostaglandin F_{2α} (PGF_{2α}) or metabolites thereof in blood or urine samples. In mammals, PGF_{2α} is produced by the uterus when stimulated by oxytocin, in the event that there has been no implantation during the follicular phase. It acts on the corpus luteum to cause luteolysis, forming a corpus albicans and stopping the production of progesterone. PGF_{2α} and its main metabolites can be found in peripheral blood and are described to be excreted mainly via the urine. There are ample tests known and described in the literature, all of which test for PGF_{2α} levels or levels of its main metabolites in blood or urine. An advantage of testing for PGF_{2α} levels is that PGF₂ₐ is identical in structure in all mammals and also its main metabolites are highly conserved among mammals. Up to now, testing for PGF_{2α} levels has only been described in animal species like human, bovine, pig, rabbit or rat. In these species the phenomenon of naturally occurring pseudopregnancy is unknown or at least extremely rare.

Drawbacks associated with known methods for testing of PGF_{2α} levels are e.g. sample acquisition. As already mentioned above, taking of blood samples is time consuming, laborious and usually requires the anesthetisation of the respective animals. The use of urine samples is also not ideal because urine is draining away in a short period of time and it is always difficult to recover enough disposed urine from a particular animal suitable for the desired measurement. Furthermore, especially if animals living in groups or herds are to be tested, it is difficult to reliably attribute a disposed urine sample to a particular animal.

It is an object of the present invention to avoid or to diminish one or more disadvantages of the state of the art. In particular, it is an object of the present invention to provide an improved method for the determination of a pregnancy status of a mammal.

### SUMMARY OF THE INVENTION:

The present invention solves said problem by the provision of a method for determination of a pregnancy status of a mammal, comprising:
i) providing faeces material of a mammal; and
ii) measuring the concentration of at least one PGF_{2α} metabolite in said faeces material.

The present invention also provides a method for the discrimination between pregnancy and pseudopregnancy of a carnivore, comprising:
i) providing blood, urine and/or faeces material of a carnivore, preferably from a carnivore of the family *felidae* and/or *canidae*; and
ii) measuring the concentration of at least one PGF_{2α} metabolite in said blood, urine of faeces material.

Basis of the present invention is the surprising finding that metabolites of PGF_{2α}, in particular of 15-keto-13,14-dihydro- PGF_{2α}, 9α,11α-dihydroxy-15-oxo-prost-5-en-1-oic acid or 9α,11α-dihydroxy-15-oxo-2,3,4,5-tetranorprostan-1,20-dioic acid, are present in faeces of mammals in a concentration sufficient to allow for reliable detection with known means.

Faeces material is easy to recover or collect without the need to anaesthetise the animal. Since faeces is usually not draining off as fast as urine, there remains more time for sample acquisition and faeces droppings are easier to attribute to a particular animal than urine dispositions. It could be shown that faeces material, in particular an alcoholic extract of faeces, is suitable as sample material to test for metabolites of PGF_{2α} by known means. The test data obtainable by the method of the invention is of high quality and allows for reliable determination of a pregnancy status in a mammal. Furthermore, it has been surprisingly found that determination of the concentration at least one PGF_{2α} metabolite in blood, urine or faeces material also allows for reliable discrimination between pregnancy and pseudopregnancy in a mammal, in particular in a carnivore like e.g. a carnivore of the family *felidae* and/or *canidae.*

The method of the invention allows for the determination of a pregnancy status of a mammal. As used herein, the term "mammal" is to be understood to refer to placental mammals and marsupials. Preferably the method is used to determine a pregnancy status of a placental mammal, more preferably of the order *carnivora,* even more preferably from a mammal of the family *felidae* and/or *canidae.*

As used herein, the term "pregnancy status" refers to the status of, preferably a female, animal and discriminates between being pregnant and being not pregnant. Pregnancy begins with successful fertilization, preferably with successful implantation of the egg. A non-pregnant status characterizes any other state of an animal in which the animal is not in a pregnant state. Especially among carnivores, in particular in the families *canidae* and *felidae,* there are a number of species that show a naturally occurring phenomenon called pseudopregnancy. The corpus luteum (the remains of an ovulated ovarian follicle) is responsible for the development of maternal behavior and lactation, which are mediated by the continued production of progesterone by the corpus luteum through some or all of pregnancy. In most species the corpus luteum is degraded in the absence of a pregnancy. However, in animals in pseudopregnant status, the corpus luteum persists in the absence of pregnancy and causes pseudopregnancy, in which the female will exhibit clinical signs of pregnancy. Pseudopregnancy is a special form of a non-pregnant state.

The method of the invention comprises the provision of faeces material derived from a mammal for which the pregnancy status is to be determined. Preferably the faeces material is derived from a placental mammal, preferably of the order *carnivora,* even more preferably of the family *felidae* and/or *canidae.* The faeces material can comprise, consist of and/or be derived from all kinds of faeces material, preferably from solid and/or wet faeces material.

The faeces material can be provided in form of an extract derived from any kind of faeces material, preferably in form of a liquid extract. The liquid extract can be an extract of an acidic solvent or solution with a pH ≤ 3. The liquid extract can be an extract of a polar solvent or solution. Preferably, the liquid extract is an alcoholic extract. Preferably the extract is an extract of a lower alkyl alcohol. More preferably the extract is an extract of C₁ to C₆ alkyl alcohols, even more preferably the extract is an extract of a C₁ to C₄ alkyl alcohol, e.g. an ethanolic or methanolic extract. Particularly suitable for use in a method of the invention are alcoholic extracts of faeces material, wherein the faeces material is extracted with ≥ 75% alcohol, e.g. ethanol or methanol, more preferably with ≥ 90% alcohol, e.g. ethanol or methanol. The alcohol can be diluted in a solvent with the required solubility with the respective alcohol in order to allow the formation of a homogenous solution, preferably the solvent is an organic solvent, more preferably the solvent is water.

Preferably solid particles are separated and/or removed from the liquid extract of faeces prior to its use in a method of the invention. Such a separation can be accomplished by any means to separate a liquid sample from remaining solid particles, provided that the separation means allow low molecular weight (i.e. less than 1 kDa) compounds to remain in the liquid phase. Examples of suitable separation techniques are centrifugation and/or filtering means.

The faeces material can be applied in the method of the invention in diluted or undiluted form. Especially if the faeces material is provided in form of a liquid extract, the faeces extract may be dilute to a desired concentration before subjecting the sample to subsequent steps of the method of the invention. The choice of the diluent and the strength of the dilution may depend on the requirements that have to be met in order to allow for proper measurement of the concentration of at least one PGF_{2α} metabolite in said faeces material and can easily be determined by a person skilled in the art.

In the method of the invention, the concentration of at least one PGF_{2α} metabolite in the faeces material is measured. Basically any metabolite that is excreted via the faeces can be measured. Preferably the at least one PGF_{2α} metabolite is a 15-keto-dihydro- PGF_{2α}, 13-keto-dinor- PGF_{2α} or 11-keto-tetranor- PGF_{2α}. More preferred the at least one PGF_{2α} metabolite is 15-keto-13,14-dihydro- PGF_{2α}, 9α,11α-dihydroxy-15-oxo-prost-5-en-1-oic acid or 9α,11α-dihydroxy-15-oxo-2,3,4,5-tetranorprostan-1,20-dioic acid.

Measurement of the concentration of at least one PGF_{2α} metabolite can be accomplished by any means that allows specific detection and quantification of the at least one PGF_{2α} metabolite. As used herein, the term "quantification" does not only refer to the exact determination of absolute amounts of a given compound, but also encompasses the determination of a given compound relative to another given compound. The term "quantification" encompasses also semi-quantification of the at least one PGF_{2α} metabolite. Means for the measurement of the concentration of at least one PGF_{2α} metabolite are well known in the art and can be provided by simple adaptation of known means by routine experimentation. Preferred methods are immunoassay methods which make use of an antibody specific for the at least one PGF_{2α} metabolite. Such immunoassay methods comprise methods that allow for the measurement of the concentration of the at least one PGF_{2α} metabolite in an ELISA (enzyme-linked immunosorbent assay) / EIA (enzyme immunoassay) format or in form of a RIA or strip assay. The skilled person is in a position to generate and develop such an immunoassay for any given PGF_{2α} metabolite without undue burden. Suitable antibodies have already been described for the PGF_{2α} metabolite being a 15-keto-dihydro- PGF_{2α}, 13-keto-dinor- PGF_{2α} or 11-keto-tetranor- PGF_{2α}. In particular antibodies specific for 15-keto-13,14-dihydro- PGF_{2α}, 9α,11α-dihydroxy-15-oxo-prost-5-en-1-oic acid or 9α,11α-dihydroxy-15-oxo-2,3,4,5-tetranorprostan-1,20-dioic acid have already been described. Examples of immunological assays such as RIA and EIA are described in Basu et al. (1987) Metabolites of PGF2 alpha in blood plasma and urine as parameters of PGF2 alpha release in cattle, Acta Vet Scand, 28:409-420; Granstrom et al. (1982) Radioimmunologic determination of 15-keto-13,14-dihydro-PGE2, Methods Enzymol, 86: 306-320; Basu et al. (1998) Radioimmunoassay of 15-keto-13,14-dihydro-prostaglandin F2alpha, Prostaglandins Leukot Essent Fatty Acids, 58:347-352; Schlegel et al. (1982) Radioimmunoassay for 13,14-dihydro-15-ketoprostaglandin F2a and its application in normo- and anovulatory woman. Acta endocrinol. 100, 98. Enzyme immunoassay methods for detection of PGF_{2α} metabolites are also commercially available.

Alternative methods for measuring the concentration of the at least one PGF_{2α} metabolite encompass mass-spectrometry methods. Examples of mass-spectrometry methods are described in Ferretti et al. (1984) Use of a Homologous Internal Standard for the Quantification of the Major Metabolite of Prostaglandin F2a in Human Urine by Multiple Ion Analysis, Anal Biochem, 136:217-222.

The method of the invention can comprise the further steps of comparing the measured concentration to a preset value and, depending on a deviation of the measured concentration from the preset value, determining a pregnancy status of said mammal. The pregnancy status of a mammal can be determined by comparing the measured concentration of at least one PGF_{2α} metabolite to a preset value that has been set for said at least one PGF_{2α} metabolite. Preferably said preset value is chosen such, that a deviation from said preset value is informative with respect to a pregnancy status of a mammal of a given species. The preset value may represent a threshold value, the exceeding of which may indicate a certain pregnancy status. Alternatively the preset value may be selected such that going below said preset value indicates a certain pregnancy status. More preferably the preset value represents or is associated with a characteristic concentration value for a particular PGF_{2α} metabolite. The preset value may be chosen such that it not only contains a certain value but also contains an information at which point in time, e.g. a certain number of days after copulation, the given value is informative on the pregnancy status of a mammal. In other words, the preset value can also encompass the information that the preset value is only informative if compared to a measured concentration for faeces material collected at, after or during a certain time interval after a particular event, like e.g. a certain number of days after copulation. The preset value may differ between different species and it may be necessary to determine the preset value for a given species before applying it in a method of the invention.

The method for determination of a pregnancy status of a mammal can be used for the discrimination between pregnancy and pseudopregnancy.

The present invention is also directed to a use of the faeces material of a mammal described above for determination of a pregnancy status of a mammal and/or for the discrimination between pregnancy and pseudopregnancy.

In the following the present invention is further described by way of examples.

### FIGURES:

- FIG. 1: shows the results of a parallelism test for PGFM standards with serially diluted different volumes of 250, 125, 62.5, 31.3, and 15 µl of lynx urine samples containing medium endogenous PGFM. Standards for PGFM were ranging from 0.02 to 10 ng/ml.
- FIG 2: shows PGFM immunoactivity within the HPLC fractions determined with PGFM EIA (black line). The elution positions of tetranor-PGF, PGF2α, and to reaffirm PGFM were estimated by LCMS analyses in separate runs. Therefore each HPLC fraction was subjected to LCMS and the analytes were quantified based on their specific transitions.
- FIG. 3: shows a HPLC-immunogram of PGF2α metabolites of urine samples from three pregnant females. Urine samples were acidified, extracted with diethylether and the extracts were subjected to HPLC separation. PGFM immunoreactivity within the HPLC fractions were determined with the PGFM EIA: Fractions 32 and 37 were used for LCMS characterization of the unknown metabolite and for PGFM identification, respectively.
- FIG. 4: shows mean prostaglandin F2α metabolite (PGFM) concentrations in pregnant (n=11) and pseudo-pregnant (n=4) Iberian lynxes. A significant divergence between the hormone levels occurs 43 days after copulation.
- FIG. 5: shows the determination of PGFM in urine collected from female Iberian lynx during four consecutive years. The animal was mated every year. In 2006 the female was mated but did not conceive (pseudo-pregnancy, white block), whereas the animal was pregnant in 2007 (3 healthy cubs after 64 days of pregnancy), 2008 (x cubs) and 2009 (x cubs, grey blocks).

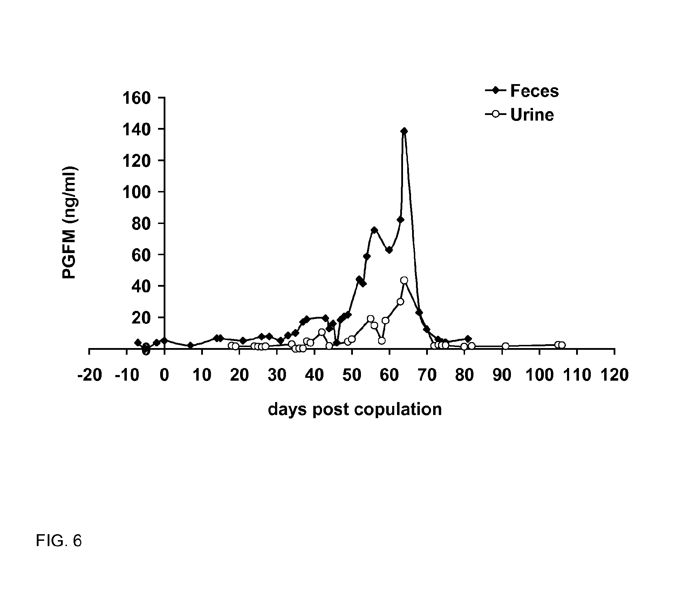

- FIG. 6: shows the course of urinary and fecal PGFM levels in samples collected from a pregnant female.

### EXAMPLES:

### Materials and methods

### Animals

The Iberian lynx Captive Breeding Center "El Acebuche" (ILCBC) in Southern Spain manages captive Iberian lynxes. The captive population consisted now of over 60 animals, half of them born in captivity. The animals are kept in separate enclosures (550 m²). All females were allowed to mate; the deliveries of cubs or abortions were final indications of pregnancy. Pseudo-pregnancies occur if fertilization failed after successful mating.

### Urine and Faeces Sampling

Urine was collected from captive lynxes by placing homemade collectors in their enclosures as described previously (Braun et al., 2009). In brief urine collectors consisted of vertical stainless steel plates (60 x 60 cm) ending in gutters at the bottom and a slight v-shape inclination that allowed the urine to run into a collector cup. Lynxes used these plates for urine-marking.

We used urine samples (variable volumes) that were collected throughout the breeding seasons in the years 2006 to 2009. Sampling started several days to weeks before the expected mating time and ended after parturition. Overall, urine samples from five pregnant, three non-pregnant and one abortive cycle were available for PGFM analysis, two pregnant females were sampled up to 5 months after parturition. Frozen samples were shipped to Leibniz-Institute for Zoo and Wildlife Research (IZW) and stored at -20° C until assayed. For the enzyme immunoassay (EIA) procedure urines were thawed, vortexed, centrifuged for 15 min at 5°C and 3000 g and an aliquot of the supernatant was diluted 1:10 with assay buffer prior to analysis.

In addition, fecal samples of one female lynx (Saliega) were collected frequently over the whole breeding season 2006 until day 80 post mating.

### Sample processing

For HPLC-immunograms and LCMS analyses of PGFM and tetranor-PGFM urine samples (0.5 ml) were acidified (pH 3) by 1 N HCL, 2.5 ml diethylether were added and the mixture was agitated for 20 minutes. Subsequently the mixture was frozen at -80°C, the ether phase was removed, evaporated under a gentle stream of nitrogen and dissolved in 150 µl 40 % methanol.

Faecal samples were processed as described herein. In brief, wet faecal samples (0.5 g) were extracted for 30 min by shaking with 4.5ml of 90% methanol. After centrifugation (15min at 3000g) the supernatant was transferred into a new tube and diluted 1:1 with water. Aliquots of the faecal extracts were subjected to the PGFM EIA.

### Stability of PGFM in urine and faecal samples

To investigate the stability of PGFM in lynx urine four samples (1 pure sample; 3 diluted 1 : 10, thereof one with the addition of 4 ng/ml PGFM) were incubated over 10 days at 37 °C. From all samples 70 µl aliquots were taken twice daily and frozen immediately at -20 °C.

To test the stability of PGFM in faeces one sample was divided into 0.5 g aliquots that were incubated over 0, 1, 2, and 3 days at 37°C each. Incubation was stopped by freezing at -20°C. At the end of the storage experiments all samples were analysed within one assay.

### Antibody and label

PGFM (9α,11α-dihydroxy-15-oxo-prost-5-en-1-oic acid) and tetranor-PGFM (9α,11α-dihydroxy-15-oxo-2,3,4,5-tetranor-prostan-1,20-dioic acid) were from Cayman Chemicals, Cayman Europe, Tallinn, Estonia, PGF2α (9α,11α, 15S-trihydroxy-(8β)-prosta-5Z,13E-dien-1-oic acid) was from Sigma, Munich, Germany. The assays were carried out with in-house microtitre plate enzyme immunoassay procedures using an PGFM antibody generated in rabbits against 9α,11α-dihydroxy-15-oxo-prost-5-en-1-oic acid-BSA as described by Schlegel et al. (Schlegel, W., Urdinola, J., Schneider, H.P.G. Radioimmunoassay for 13,14-dihydro-15-ketoprostaglandin F2a and its application in normo- and anovulatory woman. Acta endocrinol. 100, 98, 1982). The antibody was a kind gift of Prof. H.H.D. Meyer, Freising-Weihenstephan, Germany). The cross-reactivities of the PGFM antibody against tetranor-PGFM and PGF2α were <0.1 %.

The PGFM-HRP conjugate was prepared using the mixed anhydride reaction including N,N-dimethylformamide (Aldrich, Taufkirchen, Germany) and 4-methylmorpholine (Merck, Darmstadt, Germany) according to Dawson, EC, Denissen, EHC, van Weemen, BK. A simple and efficient method for raising steroid antibodies in rabbits. Steroids 31, 357-66, 1978. After the addition of 5 mg of NaHCO₃, the product was dialyzed (dialysis sack, Servapor Nr. 44145) with chromatography buffer over night and centrifuged at 5000 g and 4 °C in Centricon-10 centrifugal concentrators (Amicon, Beverly, USA). Thereafter the tubes were washed 7 times with 1 ml chromatography buffer and the label was dissolved in 1 ml assay buffer (1 % BSA). The HRP-PGFM conjugate obtained in this manner was dissolved in 1 ml assay buffer (1 % BSA), immediately tested for its titre and stored at -80°C. A two dimensional titre determination for optimum dilution of each PGFM-HRP conjugate and PGFM antiserum was carried out. Antibody dilutions ranging from 1:500 to 1:32000 and PGFM-HRP dilutions of 1:1000 to 1:23.000 were tested. The antibody titre of 1:2000 and the PGFM-HRP conjugate titre of 1:12.000 were found to be optimum in assay buffer, and an OD450 of around 1.4 was achieved by using these titers. The PGFM calibration standards were prepared by dilution with assay buffer and ranged from 0.4 to 200 pg/well. The sensitivity of the assay was defined as two standard deviations from the signal given by the zero blank. Precision and reproducibility were calculated from multiple measurements of pooled samples.

### EIA procedure

Microtiterplates were coated with goat anti-rabbit IgG (4°C, over night), blocked with BSA in coating buffer and after 30 min at 22°C the excess of blocking buffer was decanted and the plates were stored at -22°C. Prior to use the plates were washed 1x with washing solution (0.05 % Tween 20) using an automated microtiterplate washer (SLT 96PW, Tecan, Crailsheim, Germany). Duplicates of 20 µl urine sample or PGFM standards prepared in assay buffer ranging from 0.02 to 10 ng/ml were simultaneously pipetted into respective wells along with 100 µl of PGFM-HRP conjugate diluted (1:8000/1:12.000) in assay buffer (50 mM Na₂HPO₄/Na₂HPO₄, 0,15 M NaCl, 0,1 % BSA, pH 7,4) with the aid of diluter dispenser. Then, 100 µl of PGFM specific antiserum diluted in assay buffer (1:1000) were added immediately to all wells except blank by a repeat dispensor. The plates were incubated over night at 4 °C.

The plates were washed four times with washing solution and incubated further in the dark for 40 min after addition of 150 µl of substrate solution per well (1.2 mM H₂O₂, 0.4 mM 3,3',5,5'-tetramethylbenzidine in 10 mM sodium acetate, pH 5.5). The reaction was stopped by the addition of 50 µl 4 N H₂SO₄ and the intensity of colour was measured at 450 nm with a 12-channel microtiter plate reader (Infinite M 200) and hormone concentrations were calculated using the Magellan software (both from Tecan, Crailsheim, Germany).

### Parallelism

To determine the parallelism between PGFM standards and endogenous PGFM in lynx urine, four urine samples from two different individuals containing high concentrations of endogenous PGFM were serially diluted. Serial dilutions of these samples produced displacement curves parallel to that produced by the PGFM standards (prepared in buffer, ranging from 0.02 to 10 ng/ml) when run in an assay.

### HPLC immunogram

A Dionex system equipped with a quaternary pump (P580), an ASI autosampler and a column oven (STH 585) was used for chromatographic separation (Dionex GmbH, ldstein, Germany). To characterize the urinary the PGF2a metabolites, 100 µl portions of the urine extract were subjected to a reversed-phase Ultrasep ES100/RP-18/6 µm HPLC column (150 mm x 4 mm, Sepserv, Berlin). The mobile phase consisted of a mix of water : acetic acid 100 : 0.02 (v : v) and acetonitrile : acetic acid 100 : 0.02 (v : v) delivered according to the following programmed gradient (v : v): 80:20 at 0 minutes, linear gradient to 70:30 at 5 minutes, linear gradient to 30:70 at 15 minutes maintain 30:70 until 20 minutes, and return to the initial condition of 80:20 at 21 minutes in preparation for the next injection. The flow rate was set at 1 ml/min and fractions of 0.33ml were collected at 20 s intervals over a period of 20 min using a FRAC-200 fraction collector (Pharmacia Biotech, Freiburg, Germany). The elution positions of authentic PGFM and tetranor-PGFM on this column had been determined in seperate HPLC runs after injection of 500 ng of PGFM and tetranor-PGFM. Thereto 10 µl aliquots of the HPLC fractions were subjected to LCMS analyses and monitored for PGFM and tetranor-PGFM as described below.

To generate the HPLC immunograms the remaining HPLC fractions were lyophilised, reconstituted in 100 µl 40 % methanol and subjected to the PGFM EIA.

### LCMS

### Liquid chromatography

A Shimadzu separation module (CBM-20A), equipped with two quaternary pumps (LC-20AD), an autosampler (SIL-20A), a degasser DGU-20AS, and a column oven (CTO-10AS VP) was used for chromatographic separation (Shimadzu Europe, Duisburg, Germany). Separation was achieved at 30°C on a Gemini C18 column (50mmx4mm, 3µm; Phenomenex, Torrance, USA). The injection volume was 20µl. The eluent was composed of water: acetonitrile : formic acid (80 : 20 : 0.1, v : v : v) with a flow rate set to 0.5 ml/min.

### Mass spectrometry

The analyses were carried out on an API3200 QTrap LC/MS/MS system with a Turbolon Spray ESI source (Applied Biosystems/MDS-SCIEX, Palo Alto, USA) using MRM equipped with a Z spray ESI interface. A NGM-11 nitrogen generator (CMC Instruments, Eschborn, Germany) was used to generate high-purity nitrogen as nebulizer, heater, curtain and collision gases. Source temperature was 500 C. The electric potential applied on the capillary was -4.5V, and the sample cone voltage was set individually for each compound. LC-MS/MS experiments were performed at a pressure of 3.9×10⁻³ mbar and a collision energy setting adapted for each compound. Prostaglandins were detected using multiple reaction monitoring (MRM) of the two most abundant product ions per analyte. Dwell times for transition were between 100 and 300 ms.

Standards of 1 or 5 ng/µl were infused (5 µl/min) into the electrospray ionization (ESI) source to optimize MS/MS-parameters. The product ion scan mode (range 300-360) in the negative-ion mode was used for spectral analysis to select parent ions. The product ion mass spectra were obtained by choosing the molecular anions as the precursor ions and scanning MS2 from m/z 100 to 400 with a scan time of 1 s.

Optimization of mass spectrometer parameters was carried out by injecting 10 µl of standards (1 or 5 ng/µl) into a carrier stream of 500 µL/min acetonitrile/water/formic acid (50/50/0.1, v/v/v) generated by the HPLC pump using the automatic optimizing for the analyte (FIA) function of the instrument.

In the selected reaction monitoring mode, the instrument monitored the m/z 353.1 to m/z 195.2, m/z 353.1 to m/z 182.9, and m/z 353.1 to m/z 113.3 transitions for PGFM, m/z 311.1 to m/z 293.1, m/z 311.1 to m/z 121.3, and m/z 311.1 to m/z 109.2 transitions for tetranor PGFM, and m/z 353.2 to m/z 309.4, m/z 353.2 to m/z 309.2, and m/z 353.2 to m/z 193.4 for PGF2α.

### Metabolite identification

To determine PGFM and possible further metabolites binding to the PGFM antibody, 10 I of the immunoreactive HPLC fractions were subjected to LCMS analysis.

### Statistical analyses

All tests were conducted using the statistical packages Instat Version 3 (Graphpad Software Inc.) and were two-tailed with P set at 0.05. Prior to statistical analysis, all data sets were tested for departures from normality. When such departures were detected, we employed the nonparametric equivalents of the appropriate parametric statistical tests. Student's paired t-test was used to assess the stability of urinary PGFM after storage and to evaluate the recovery of added amounts of PGFM to urine.

### Results

### Test criteria of the PGFM assay

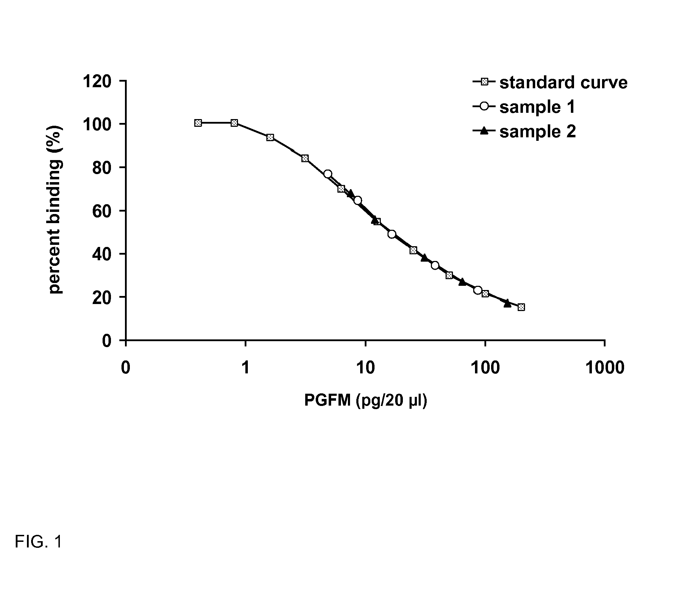

The detection limit of the assay was 0.4 pg/well. To demonstrate parallelism, two urine samples with medium concentration of endogenous PGFM were serially diluted with assay buffer and measured against the standard curve prepared in assay buffer. Displacement curves parallel to those of the PGFM standard were obtained (FIG. 1).

The intra- and inter-assay coefficients of variation (CV) were determined by using one urine sample (1:10 diluted in assay buffer) and buffer pools containing low (0.15 ng/ml) and high (4 ng/ml) concentrations of PGFM. n (Intraassay) = 10, n (Interassay) = 45. The results are presented in Table 1.

**Table.1: Precision of PGFM determination in urine**

| **Quality control** | **Mean (ng/ml)** | **Intra-assay CV (%)** | **Inter-assay CV (%)** |
|---|---|---|---|
| Low (in assay buffer) | 0.15 | 10.4 | 14.3 |
| High (in assay buffer) | 4.00 | 7.6 | 7.4 |
| Sample | 0.69 | 6.3 | - |

The recovery of added PGFM was determined by measuring PGFM previously added to diluted lynx urine with low endogenous PGFM concentration (0.136 ng/ml). Additions of 0.3, 0.6, 1.25, 2.5 and 5.0 ng/ml PGFM were recovered as 0.33, 0.53, 1.14, 2.87 and 6.65 ng/ml, respectively. The correlation coefficient of added and measured hormone concentration was significant (r = 0.997; P < 0.001). The overall recovery of added PGFM was 119%.

### Stability of PGFM in lynx urine

To investigate the stability of PGFM in samples of lynx urine, samples containing 1.80, 1.85, 3.30 and 3.7 ng/ml were incubated at 37°C up to 10 days and subjected to measurements. The results revealed no significant decrease (P>0.05) of PGFM within a 10 days storage period, indicating that PGFM in urine is stable even at elevated temperature for at least 10 days. Compared to the initial concentrations the recoveries after 10 days of storage were 1.50 ng/ml (83 %), 2.95 ng/ml (159 %); 2.90 ng/ml (88%) and 4.10 ng/ml (111 %). The overall recovery was 110 %.

### Identification of PGFM in urine of the Iberian lynx

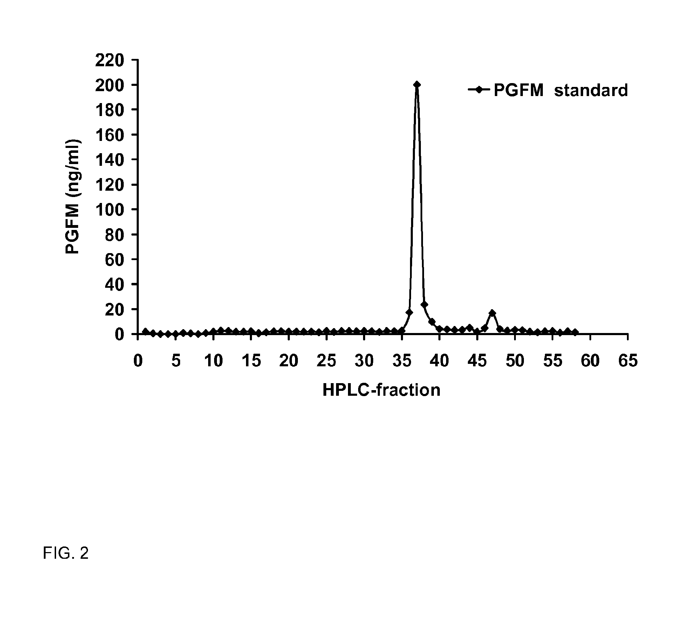

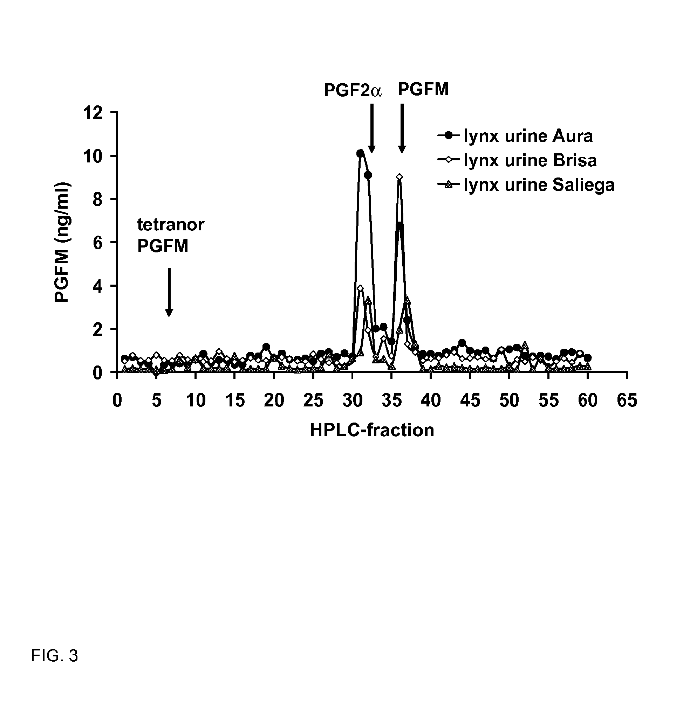

For separation and characterization of PGFM and possible additional PGF2α metabolites, HPLC immunograms of urinary extracts from three animals were developed (FIG. 3). The elution positions of compounds with antibody binding capacities (immunoreactivities) were determined in the PGFM immunoassay, the elution positions of tetranor-PGFM (fraction 6), PGF2α (fraction 32) and PGFM (fraction 38) had been determined previously in separate HPLC runs (injection of 500 ng per standard) and by analysis of the HPLC fractions in LCMS and PGFM EIA (FIG. 2). The PGFM standard peaked in fraction 38, followed by a minor immunoreactivity in fraction 48. Due to missing cross-reactivities of tetranor-PGFM and PGF2α with the PGFM antibody their detection demands LCMS analysis demonstrating them in fractions 6 and 32, respectively. In addition PGFM was confirmed in fraction 38 exactly matching with the PGFM immunoreactivity.

To investigate whether lynx urine might contain tetranor-PGFM HPLC fraction 6 (FIG. 3), supposed to contain tetranor-PGFM, was subjected to LCMS analyses. Here tetranor-PGFM was clearly detectable based on its specific transitions m/z 311.1 to m/z 293.1, m/z 311.1 to m/z 121.3, and m/z 311.1 to m/z 109.2.

To confirm the identity of PGFM fraction 37 (FIG. 3) containing its highest amount was also subjected to LCMS analyses. Here, PGFM could be clearly authenticated based on its specific transitions m/z 353.1 to m/z 195.2, m/z 353.1 to m/z 182.9, and m/z 353.1 to m/z 113.3.

### PGFM profiles in pregnant and pseudo-pregnant of Iberian lynxes

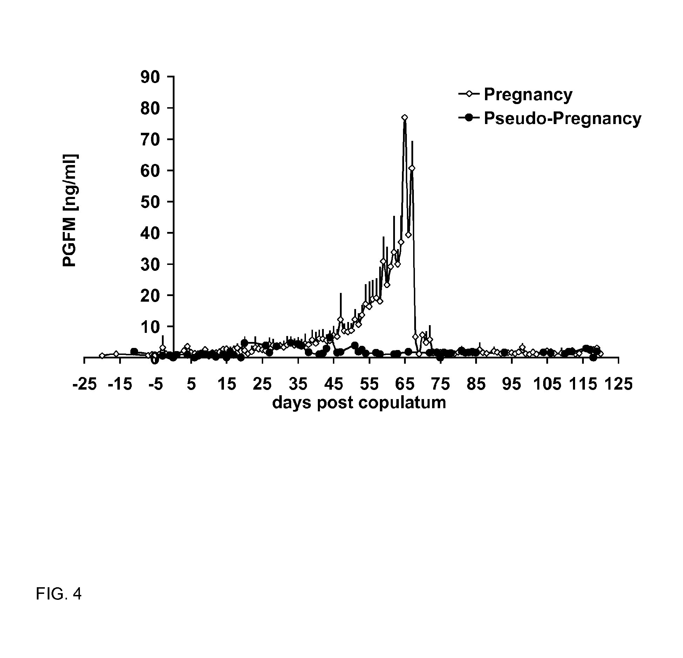

Urinary PGFM concentrations were estimated in samples collected from 20 days prior mating and ending several weeks after parturition. The temporal changes in the mean levels of PGFM from 11 pregnancies are presented in FIG. 4. Urinary PGFM levels (mean ± S.D.) were basal (1.5 +- ng/ml; mean ± S.D.) prior to mating and remained there until day 15 post copulation (p.c.) followed by a gradual elevation to levels of about 7 ng/ml. Day 30 marked the beginning of an exponential increase culminating in peak levels of 76.9 ng/ml (76.9 +- SEM ng/ml) at day 65 p.c. Postpartum PGFM concentrations immediately decreased to a basal (1.66 +- SEM ng/ml) and remained low over the rest of the sampling period.

By contrast the four pseudo-pregnant female deviate from the pregnancy-related course after day 36. Urinary PGFM concentrations showed a transient increase to 7 ng/ml on day 41. Thereafter the course of the non-pregnant females definitely separates from those of the pregnant females dropping to basal on day 43 p.c. Thus, it is preferred to use PGFM measurements for pregnancy diagnosis in the Iberian lynx from day 43 p.c.

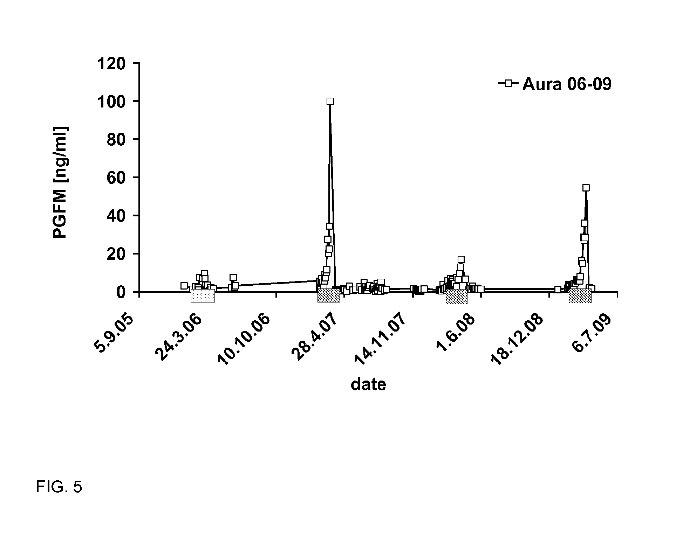

From one female urine samples were collected over a period of four successive years including all mating periods but with interruptions during the non-breeding season (FIG. 5). The profile shown in FIG. 5 revealed a pseudo-pregnancy followed by three pregnancies during the subsequent years. A sample collection gap of some days occurred during the second pregnancy and might have led to a skip of the peak values during second pregnancy wrongly insinuating lower peak concentrations during this pregnancy.

### Identification of PGFM in faeces of the Iberian lynx

Urinary and fecal PGFM concentrations determined in samples collected from the same animal are shown in FIG. 5. Analyses of PGFM in both matrices revealed similar courses leading to peak levels in urine and feces of 45 ng/ml and 135 ng/ml after 63 and 64 days of pregnancy, respectively. In feces, however, an increase above baseline levels seems to occur earlier on day 35 of pregnancy. Based on 14 fecal and urine samples obtained from the same day a highly significant correlation between both determinations was obtained (r² = 0.927, p < 0.001). In summary, faeces material is at least equally well suited as starting material for the methods of the invention described above, in particular for determination of a pregnancy status of a mammal, compared to urine samples.

## Claims

1. Method for determination of a pregnancy status of a mammal, comprising:
i) providing faeces material of a mammal; and
ii) measuring the concentration of at least one PGF_{2α} metabolite in said faeces material.

2. Method of claim 1, wherein the faeces material is derived from a mammal of the order *carnivora,* preferably from a mammal of the family *felidae* and/or *canidae.*

3. Method for the discrimination between pregnancy and pseudopregnancy of a carnivore, comprising:
i) providing blood, urine and/or faeces material of a carnivore, preferably from a carnivore of the family *felidae* and/or *canidae*; and
ii) measuring the concentration of at least one PGF_{2α} metabolite in said blood, urine of faeces material.

4. Method of one of the preceding claims, wherein the at least one PGF_{2α} metabolite is a 15-keto-dihydro- PGF_{2α}, 13-keto-dinor- PGF_{2α} or 11-keto-tetranor- PGF_{2α}.

5. Method of one of the preceding claims, wherein the at least one PGF_{2α} metabolite is 15-keto-13,14-dihydro- PGF_{2α}, 9α,11α-dihydroxy-15-oxo-prost-5-en-1-oic acid or 9α,11α-dihydroxy-15-oxo-2,3,4,5-tetranorprostan-1,20-dioic acid.

6. Method of one of the preceding claims, wherein the faeces material is a liquid extract of faeces.

7. Method of one of the preceding claims, wherein the faeces material is an alcoholic extract of faeces.

8. Method of one of the preceding claims, wherein the faeces material is a lower alkyl alcohol extract of faeces, preferably an extract of ≥ 75% lower alkyl alcohol, more preferably an extract of ≥ 90% lower alkyl alcohol.

9. Method of one of the preceding claims, wherein the faeces material is a liquid extract prepared by separating the liquid extract from solid particles.

10. Method of one of the preceding claims, wherein the concentration of at least one PGF_{2α} metabolite in said faeces material is measured by an ELISA, EIA, RIA, strip assay or by a mass-spectrometry method.

11. Method of one of the preceding claims, further comprising
iii) comparing the measured concentration of the at least one PGF_{2α} metabolite to a preset value; and
iv) depending on a deviation of the measured concentration from the preset value, determining a pregnancy status of said mammal.

12. Use of a method of one of the preceding claims for the discrimination between pregnancy and pseudopregnancy.

13. Use of faeces material of a mammal for determination of a pregnancy status of a mammal.

14. Use of faeces material of a mammal for the discrimination between pregnancy and pseudopregnancy.
